# EUROPEAN PATENT APPLICATION

(11) **EP 3 892 713 A1**
(43) Date of publication of application: **13.10.2021**
(21) Application number: 20168778.7
(22) Date of filing: 08.04.2020
(51) Int. Cl.: C12M 1/32, C12M 3/06, C12M 1/00, B01L 3/00, C12N 5/00

(54) **MULTI-WELL PLATES FOR CREATION OF HIGH-DENSITY ARRAYS OF COMPARTMENTALIZED CELL CULTURES FOR USAGE IN HIGH CAPACITY APPLICATIONS**

(71) Applicant: CELLECTRICON AB, 431 53 Mölndal (SE)
(72) Inventor: PIHL, Johan, 424 70 Olofstorp (SE); KARLSSON, Mathias, 439 94 Onsala (SE); KARILA, Paul, 427 50 Billdal (SE)
(74) Representative: Adam, Holger

(57) **Abstract**

The disclosure relates to multi-well plates having fluidic connections between neighboring wells that are useful to produce a cell culture substrate and compliant with American National Standards Institute of the Society for Laboratory Automation and Screening (ANSI/SLAS) microplate standards.

## Description

### Field

The present disclosure relates to novel substrates for generation of high-density arrays of compartmentalized cell cultures for usage in rational drug discovery applications. Specifically, in some embodiments, the disclosure relates to an Society for Laboratory Automation and Screening (ANSI/SLAS) microplates standard compliant multi-well plate wherein groups of wells are fluidically connected to produce a cell culture substrate that can be used for a wide range of axonal transport assays in neurobiology research and drug discovery.

### Background

Since its conception in the mid 1970's, compartmentalized cell cultures (CCC) have gained traction as an important methodology in neurobiology research. Campenot, R.B., Proc. Natl. Acad. Sci. U. S. A. 74: 4516-9 (1977). For example, CCC's can be used to study network communication between discrete population of neurons to study mechanisms such as synaptic communication (Vikman et al., J. Neurosci. Methods 105:175-184 (2001)), axonal transport of proteins and organelles (Bousset et al., Ann. Neurol. 72:517-524 (2013), or for the purpose of studying cell-network formation (Taylor et al., J. Neurosci. 33:5584-5589 (2013)). In addition, CCC's are being used for experiments to study cellular signaling between different cell types, for example neuron - muscle cell signaling (Zahavi et al., J. Cell Sci. 128:1241-1252 (2015)), or communication between neurons from different brain regions (Berdichevsky, Y., Staley, K. J. & Yarmush, M. L. Lab Chip 10, 999-1004 (2010).).

Traditionally, CCC's have mainly been used for basic research application where there has been limited need for high throughput or parallelization of experiments. However, because of the increasing demands from the pharmaceutical industry for more advanced and translationally relevant cell-based assay, there is now a need of being able to use CCC's in drug screening applications. For example, there is today a big interest to gain access to assay platforms that, in a relevant manner, can model prion- and prion-like mechanisms and enable screening of thousands of compounds in relatively short time frames (Zhang, M., Luo, G., Zhou, Y., Wang, S. & Zhong, Z. Phenotypic screens targeting neurodegenerative diseases. J. Biomol. Screen. 19, 1-16 (2014).). However, current state-of-the-art products cannot provide sufficient robustness or throughput to meet such demands.

To establish CCC's, cell culture substrates are being employed where discrete cell culture regions (wells) are fluidically interconnected through extremely small tubes with a diameter sufficiently large to establish a fluidic connection between the wells but sufficiently small to prevent cells to migrate between different cell-culture regions. Traditionally, CCC's were achieved through manual and very crude means: using a scalpel, grooves or scratches were manually made in the bottom of a cell culture dish. The scratches were then sealed using vacuum-grease, and discrete regions were then formed by careful positioning of a physical barrier such as glass or polytetrafluoroethylene (PTFE) rings on top of the sealed scratches. For a description of this method, see Campenot, R.B., Proc. Natl. Acad. Sci. U. S. A. 74: 4516-9 (1977). Although this method can be used to produce substrates suitable for formation of CCC's it is very cumbersome and plagued by a high failure rate. In recent years, micromachining methods have been used for production of substrates for CCC formation. For example, soft lithography and polydimethylsiloxane (PDMS, i.e. silicone rubber) casting have been employed to produce microfluidic devices that are highly uniform and much easier to handle than the original handmade substrates. See Taylor et al., Nat. Methods 2:599-605 (2005); and Neto et al., J. Neurosci. 36:11573-11584 (2016). However, due to the rather complex microchannel networks required to enable formation of CCC's, also these microfluidic devices display several drawbacks that prevents them for being used for efficient experimentation. For example, these devices are difficult to fill with liquids, are prone to bubble formation, are difficult to surface modify, cause sheer stress on the cell-cultures, prevent free diffusion of gas and nutrients to the cells, and are prone to delamination over time. To remedy the cell-culture related issues associated with microfluidic devices as described above, there are reports in literature of a simplified device design composed of two large sized (5x5 mm) open cell culture wells directly interconnected by small microfluidic channels. Similar to the microfluidic devices described above, also this device was produced in PDMS using a soft lithography method. By using this type of device, it was possible to culture certain types of neurons that will not survive in a typical microfluidic substrate (Lu et al, J. Neurosci. Methods 209, 35-39 (2012)). Although all the micromachined devices described above provide a great improvement over the original Campeont chamber design, it is evident that they are plagued by several drawbacks. In addition to the problems already discussed above, it is also impossible to scale-up these designs into a high-density format required for high-capacity screening because of the large sized wells and complex microchannel layouts required for these substrates. Also, these micromachined substrates are normally manufactured in soft polymeric materials such as PDMS which exhibit flaccid and elastic material properties making such materials unsuitable for production of devices that require tight spatial tolerances such as a screening microtiter plate. Finally, these micromachined devices does not provide an ideal optical environment for the type of high-resolution imaging that is required for monitoring axonal transport. Typically, analysis of axonal transport requires imaging of neurites (cellular processes) residing inside the fluidic connections in order to measure the linear translocation of target proteins and organelles. Accordingly, it is essential that the fluidic connections do not provide differential optical conditions such as variable contrast, refraction index, or light emittance or transparency. In the case of automated imaging, the fluidic connections must be positioned at coordinates that an imaging instrument can access, i.e. a fluidic connection residing in a wall between two wells will often not be accessible for an automated imaging instrument. In summary, there is a need for designs and production methods that enables creation of a microfluidic substrate in the form of an ANSI/slas compliant microtiter plate that can provide a high-density array of CCCs for drug screening applications.

### SUMMARY

Here we present a novel substrate that enables the formation of high-density arrays of CCC's that enable high capacity experimentation applications such as medium- and high-throughput screening (MTS and HTS respectively). The substrate is based on, but not limited to, a standard 384-well plate format wherein at least two wells in the plate are interconnected by fluidic connections in the form of closed channels that can be made sufficiently small to prevent migration of cells, and even to maintain chemical integrity between wells and where said channel(s) is located essentially in the plane of the bottom of the well. Said microtiter plate is thus comprising open wells and has an optically transparent bottom and whose surface properties enables culturing of cells. The fluidic connections have been carefully designed to enable robust liquid handling to ensure high success rates in experiments, also the substrate can easily be surface modified using wet-chemical approaches. Furthermore, the substrate is designed to provide optimal conditions for high-resolution imaging through careful selection of substrate materials, and design and placement of fluidic connections. In order to enable usage in HTS applications, the substrate has been designed to obey all ANSI/SLAS microplate standards and is therefore compatible with most commercially available liquid handling robotics and optical readout systems available on the market.

In accordance with the description, the present disclosure encompasses, for example, a multi-well plate comprising wells, wherein at least two adjacent wells in the plate have at least one fluidic connection trough the wall separating the at least two neighboring wells and where said fluidic connection is located essentially in the plane of the bottom of the well. In some embodiments, the present disclosure encompasses, for example, a multi-well plate comprising wells, wherein at least two adjacent wells in the plate have at least one fluidic connection and where said fluidic connections partially traverse at least one well in the multi-well plate. In some embodiments, the present disclosure encompasses, for example, a multi-well plate comprising wells, wherein at least two non-adjacent wells in the plate have at least one fluidic connection and where said fluidic connections completely traverse a well adjacent to at least one of said non-adjacent wells and where said adjacent well also is equipped with optical detection markers in the same plane as the at least one fluidic connection to enable a microscope or high-content imaging system to autofocus in said plane. In some embodiments, the multi-well plate complies with American National Standards Institute of the Society for Laboratory Automation and Screening (ANSI/SLAS) microplate standards. In some embodiments, the multi-well plate comprises a substrate produced from a thermoplastic material to facilitate high precision manufacturing. In some embodiments, the thermoplastic material comprises polystyrene (PS), cyclo-olefin-copolymer (COC), cycloolefin polymer (COP), poly(methyl methacrylate (PMMA), polycarbonate (PC), polyethylene (PE), polyethylene terephthalate (PET), polyamide (Nylon®), polypropylene or polyether ether ketone (PEEK), Teflon®, PDMS, and/or thermoset polyester (TPE). In some embodiments, the multi-well plate comprises a substrate produced from cyclo-olefin-copolymer (COP), cyclo-olefin-polymer (COC) or polystyrene (PS). In other embodiments, the multi-well plate comprises a substrate produced from silicon, glass, ceramic material, or alumina. In some embodiments, the plate comprises a substrate comprising more than one layer, optionally wherein the layers are bonded by ultrasonic welding, thermocompression bonding, plasma bonding, solvent-assisted bonding, laser-assisted bonding, or adhesive bonding using glue or double adhesive tape. In some embodiments, the plate comprises a substrate coated with a protein or polymer. In some cases, the plate comprises a substrate coated with one or more of poly-l-lysine, poly-L-ornithine, collagen, laminin, Matrigel®, or bovine serum albumin. In some cases, the plate comprises a substrate comprising a surface chemically modified with one or more of poly[carboxybetaine methacrylate] (PCBMA), poly[[2--methacryloyloxy)ethyl]trimethylammonium chloride] (PMETAC), poly [poly(ethylene glycol) methyl ether methacrylate] (PPEGMA), poly[2-hydroxyethyl methacrylate] (PHEMA), poly[3-sulfopropyl methacrylate] (PSPMA), and poly[2-(methacryloyloxy)ethyl dimethyl-(3-sulfopropyl)ammonium hydroxide] (PMEDSAH).

In some embodiments, the plate comprises at least 32, or at least 96 groups of three fluidically connected wells. In some embodiments, the at least one fluidic connection comprises cross-sectional dimensions of at least 1-10 µm and at most 10-20 µm, optionally with an aspect ratio ranging from 1:5 to 2:1 (height:width). In some embodiments, the at least one fluidic connection comprises cross-sectional dimensions (H and/or W) between 1-20 µm, such as 1-5 µm, 5-10 µm, 10-15 µm, 15-20 µm, 5-15 µm, or comprising cross-sectional dimensions (H and/or W) of 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, or 20 µm, and optionally also having an aspect ratio (H x W) ranging from 1:5 -2:1. In some embodiments, the fluidic connection comprises cross-sectional dimensions of equal to or less than 5 x 5 µm, or of 3 x 3 µm to 5 x 5 µm. In some embodiments, the dimensions, shape and number of fluidic connections are varied across the length of the at least one fluidic connection to improve neurite penetration and producibility. In some embodiments, the length of the at least one fluidic connection is at least 0.5 mm and at the most 5.0 mm. In some embodiments, the aspect ratio of the dimensions of the at least one fluidic connection ranges from 20:1 (W:H) to 1:5 (W:H).

In some embodiments, the multi-well plate comprises a 96, 384, 1536 or 3456 well format, and is optionally organized in a 2:3 rectangular matrix. In some embodiments, the multi-well plate comprises at least 2 groups of three neighboring and fluidically interconnected wells. In some embodiments, the multi-well plate comprises at least 3 groups of two neighboring and fluidically interconnected wells. In some embodiments, the multi-well plate comprises at least 1 group of four neighboring and fluidically interconnected wells.

The present disclosure also encompasses methods for high throughput screening of a material of interest, comprising screening the material of interest using the multi-well plate of any one of the embodiments herein. In some embodiments, the material of interest is a 2D cell culture. In other embodiments, the material of interest is a 3D cell culture.

Additional objects and advantages will be set forth in part in the description which follows, and in part will be obvious from the description, or may be learned by practice. The objects and advantages will be realized and attained by means of the elements and combinations particularly pointed out in the appended claims.

It is to be understood that both the foregoing general description and the following detailed description are exemplary and explanatory only and are not restrictive of the claims.

The accompanying drawings, which are incorporated in and constitute a part of this specification, illustrate one (several) embodiment(s) and together with the description, serve to explain the principles described herein.

### BRIEF DESCRIPTION OF THE DRAWINGS

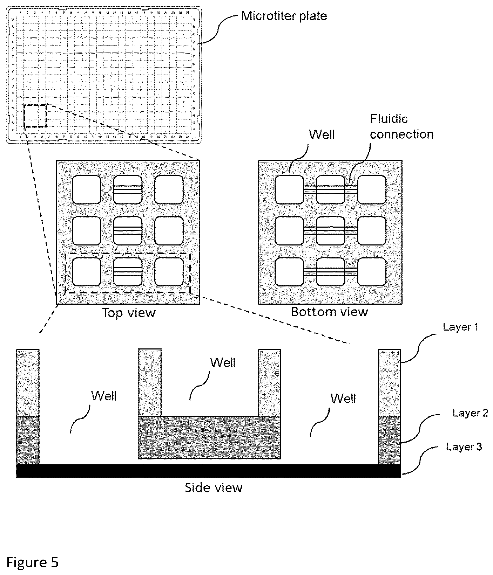

Figure 1 provides an illustration of one method of fabrication and assembly of the microplate. The two layers (layers 2 and 3) are bonded forming a laminate to seal and define the fluidic connections. The illustration shows the combined laminate (layers 2 and 3) bonded to bottomless 384-well plate (layer 1). Layer 2: Thick, +1mm substrate with milled through holes matching 384-well plate pattern containing trenches on underside
   Layer 1: Standard 384-well plate without bottom
   Bottom part (layer 3): thin transparent and preferably HCA compatible sheet
Figure 2 illustrates the substrate containing the connections between wells, and exemplifies two possible fluidic connections between said wells.
Figure 3 shows how different designs, i.e., pair coupled wells, three connected wells and four-connected wells can be packed in a microplate format.
Figure 4 shows a side view of the substrate, and how this can be assembled in a three-layer as well as in a two-layer design.
   The following is shown in Fig. 4A:
   Layer 1: standard top-part from 384-well microtiter plate;
   Layer 2: Thick, +1mm substrate with milled through holes matching 384-well plate pattern containing trenches on underside;
   Layer 3 -Plate bottom, i.e. thin film (100-200 µm) bonded to above layer 2.
   The following is shown in Fig. 4B:
   Layer 1 - standard top-part from 384-well microtiter plate
   Layer 3 -Plate bottom, i.e. thin film (100-200 µm) bonded to above layer 2
Figure 5 illustrates a substrate specifically designed for studying axonal transport processes in real-time using a high-resolution imaging system. In this design, two non-adjacent wells are fluidically connected and the fluidic connection is traversing a well situated between the fluidically connected wells. This design requires a three-layered substrate.

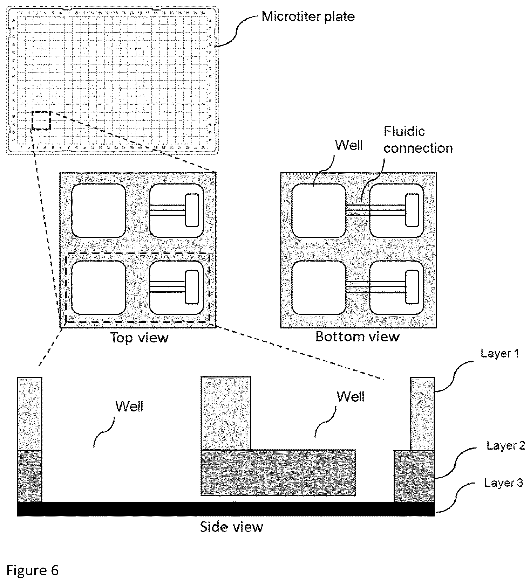

Figure 6 illustrates a substrate specifically designed for studying axonal transport in real-time using a high-resolution imaging system. In this design, two adjacent wells are fluidically connected and the fluidic connection is allowed to partially traverse one well in order to facilitate imaging inside the fluidic connection in a position that provides ideal optical conditions. This design requires a three-layered substrate.

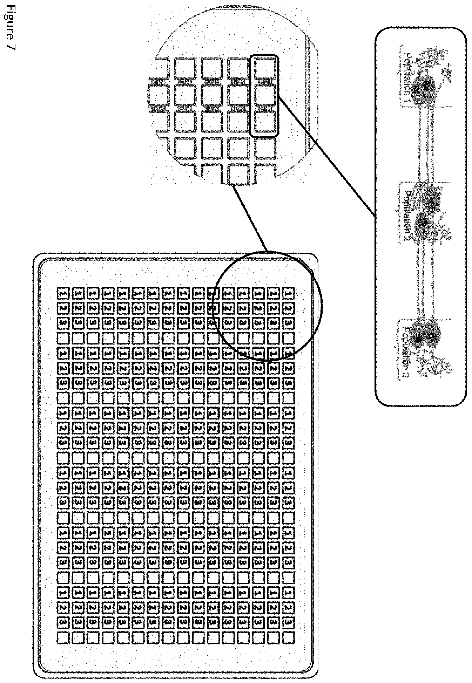

Figure 7 illustrates a prion progression and modulation assay concept. A prion-like mechanism inducer (e.g. pathogenic Tau) is added to well one, the progression of pathogenesis is then modulated in wells 2, and the modulation can be detected in well 3.

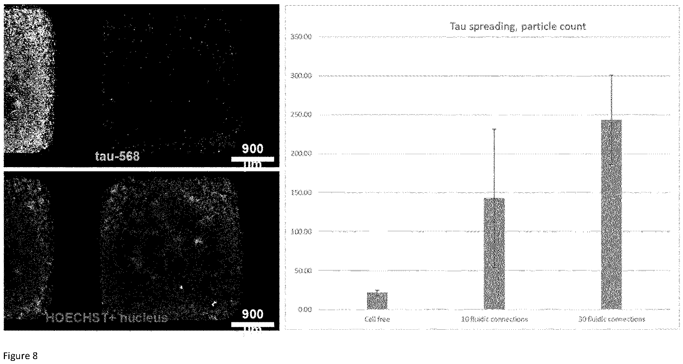

Figure 8 shows microscopy images of spread of fluorescently labelled NDAPs (Tau particles) between cells cultures in neighboring wells connected by fluidic connections. The graph further demonstrates that spreading is dependent on the number of fluidic connections, and that cells are required for transport between wells.

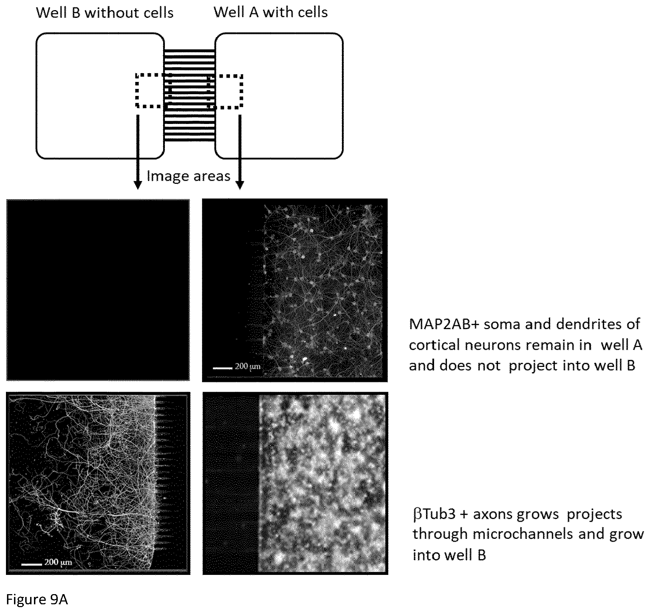

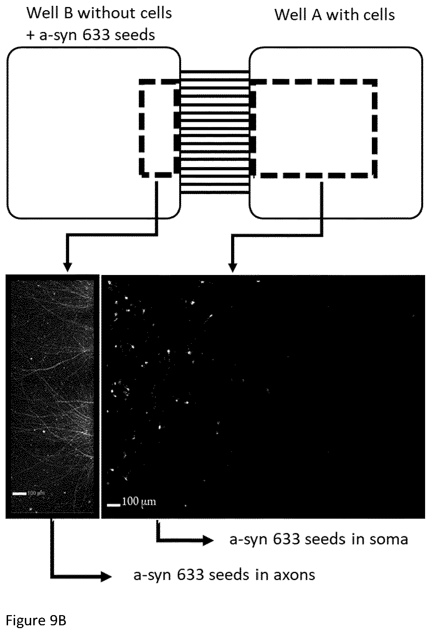

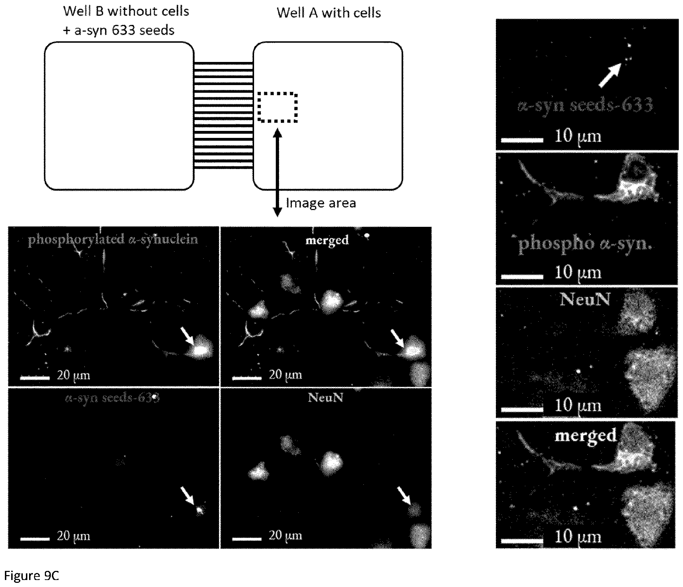

Figure 9 shows data from an experiment where retrograde axonal transport of alphasynuclein oligomers was investigated.

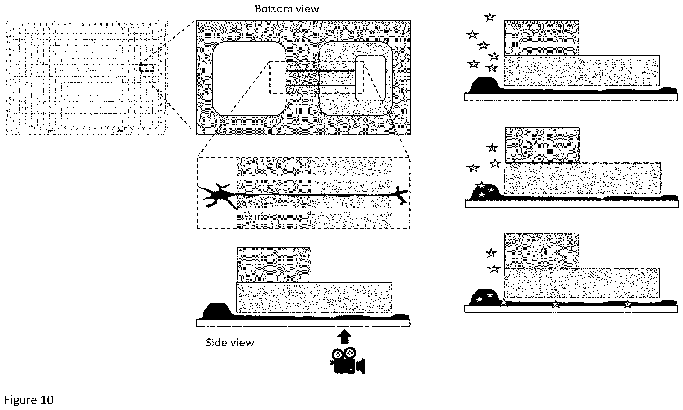

Figure 10 shows a concept for assaying axonal transport in primary neurons using dynamic imaging and a specialized substrate outlined in Figure 6.

### DESCRIPTION OF CERTAIN EMBODIMENTS

The present disclosure relates to a novel substrate for generation of high-density arrays of compartmentalized cell cultures (hereinafter referred to as CCC) for usage in high capacity applications, such as medium and high throughput screening (hereinafter referred to as MTS and HTS respectively). Specifically, the disclosure relates to an ANSI/SLAS standard, compliant multi-well plate, which, in some embodiments can be a 384-well plate, wherein groups of wells are fluidically connected through microfabricated closed channels that are sufficiently small to prevent migration of cells or clusters of cells and/or to maintain chemical integrity between wells.

### Definitions

As used herein, the term "about" refers to a numeric value, including, for example, whole numbers, fractions, and percentages, whether or not explicitly indicated. The term about generally refers to a range of numerical values (e.g., +/-5-10% of the recited range) that one of ordinary skill in the art would consider equivalent to the recited value (e.g., having the same function or result). When terms such as at least and about precede a list of numerical values or ranges, the terms modify all of the values or ranges provided in the list. In some instances, the term about may include numerical values that are rounded to the nearest significant figure.

The term "or" as used herein should be interpreted as "and/or" unless otherwise made clear from the context that only an alternative is intended.

A "multi-well plate" refers to a flat plate having wells or compartments that can be utilized as small test tubes. The multi-well plate can have, in some examples, 96, 384, 1536 or 3456 wells organized, in some embodiments, in a 2:3 rectangular matrix. A "substrate" of a plate refers to the general materials forming the plate structure and wells of the plate. A substrate can comprise one or more layers as well as one or more coatings.

A "384-well format" refers to a multi-well plate having 384 wells organized in a 2:3 rectangular matrix, i.e., 16x24 wells. In this context, the term "format" merely refers to the way in which the rows of wells are organized (e.g., 2:2, 2:3, and the number of wells in each row, that provides the total number of wells). Higher multi-well plate formats (1536-wells), for instance, having 32x48 wells or lower multi-well plate formats (96-wells) having 8x12 wells can also be used. The plate formats envisioned could be, for example, 96, 384, 1536 or 3456 wells.

The terms "connected wells," or "interconnected wells" or "fluidically connected wells" refer to wells having direct fluidic connections between them. "Neighboring wells" refer to adjacent wells and may be interconnected by one or several fluidic connections, forming so called "groups" of wells. "Groups" of wells, as used herein, refers to wells connected directly or indirectly by fluidic connections. In some embodiments, such groups of wells may form "assayable structures" or "assayable entities" or "assayable groups," i.e., structures or entities used for an intended assay. A group of at least 3 interconnected wells, for example, may form an assayable entity. Such groups of wells may be addressed individually or in multiple groups in parallel or sequentially on the 384 well plate.

The term "fluidic connection," such as between wells, refers to wells having one or more connection or conduit, which depending on the purpose can allow for controlled transport or the prevention of transport of materials. In this embodiment, a fluidic connection is composed of one or several micromachined closed channels that connects two wells in a multiwell plate. In one embodiment, the fluidic connection allows growth/transport of axons and/or dendrites but prevents transport of cells or cell bodies. In this embodiment, small molecules, polymers, proteins and nanoparticles can be transported through the fluidic connections between wells and said transport can be modulated by manipulating the hydrostatic pressure. In another embodiment, the fluidic connection allows for transport of cells, clusters of cells as well as axons and dendrites. The term "cross-sectional dimensions" refers to the width and height of the fluidic connection between two wells.

The "Society for Laboratory Automation and Screening (ANSI/SLAS) microplate standards" refers to a set of standards that outlines physical dimensions and tolerances for footprint dimensions, height dimensions, outside bottom flange dimensions, well positions and well bottom elevation elevations. For example, in some embodiments, the multi-well plate complies with valid ANSI/SLAS standards, namely the ANSI/SLAS 1-2004 (R2012): Footprint Dimensions, ANSI/SLAS 2-2004 (R2012): Height Dimensions, ANSI/SLAS 3-2004 (R2012): Bottom Outside Flange Dimensions, ANSI/SLAS 4-2004 (R2012): Well Positions, and/or ANSI/SLAS 6-2012: Well Bottom Elevation.

The term "thermoplastic material" refers to a plastic material, most commonly a polymeric material, that becomes moldable or pliable above a certain temperature, and solidifies upon cooling.

### Compositions and Methods

### A. Substrate Characteristics

In order to meet the current requirements for commercial medium- and high-throughput screening (MTS-HTS) systems, the substrate of the present disclosure, in some embodiments, may have a physical footprint and outer shape as specified in the ANSI/SLAS microplate standards. By obeying those standards, embodiments of the present disclosure may be compatible with established robotic plate handling systems, liquid handling systems, and optical readout systems utilized in MTS-HTS. However, as standards for microplates are subject to future changes in shape or design, the present invention is also compatible with a variety of shapes and sizes of multi-well plates.

In one embodiment of the disclosure, the substrate is composed of three parts:

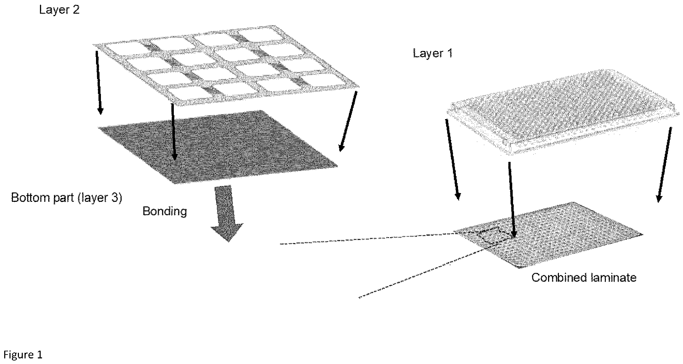

First, the substrate is composed a top part that defines the outer dimensions and shape of the substrate. Also, the first part defines the macroscopic part of the wells or cell culture regions. The size and geometry of these wells should be designed to facilitate liquid handling and cell-culture processes. In one embodiment of the present disclosure, a 384-well format is used. However, in other embodiments of the disclosure, multi-well plates having 96, 1536 or 3456 wells can be used. (Fig. 1). For example, in embodiments that use a 384 well plate, the plate comprises at least 96 groups of three neighboring and fluidically interconnected wells, at least 192 groups of two neighboring and fluidically interconnected wells, or at least 96 groups of four neighboring and fluidically interconnected wells. Multi-well plates with 96, 1536 or 3456 wells having groups of two or three interconnected wells could also be used.

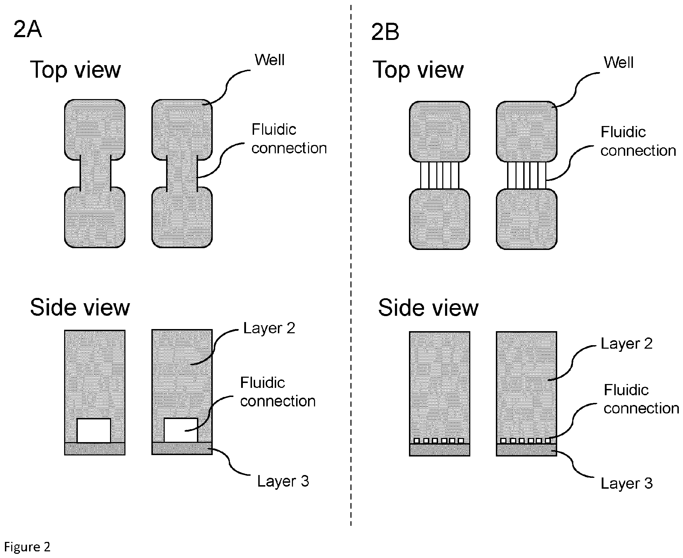

The second and middle part of the substrate defines the fluidic connections between wells. (Fig. 1) Depending on the application, the size and length of these fluidic connections can be varied and depends on the type of cell-based assay where the substrate is to be used. For assaying axonal transport mechanisms, such as prion-like spreading of neurodegenerative disease associated peptides, connections sufficiently small to prevent cellular migration of cells between different cell-culture zones (wells). (Fig. 2). The cross-sectional dimensions of the fluidic connections may in this case comprise one or more connections having a dimension between 1-20 µm, such as 1-5 µm, 1-10 µm, 5-10 µm, 10-20 µm, 10-15 µm, 15-20 µm, 5-15 µm, or having a dimension (H or W) of 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, or 20 µm, and optionally also having an aspect ratio (H x W) ranging from 1:5 - 2:1.

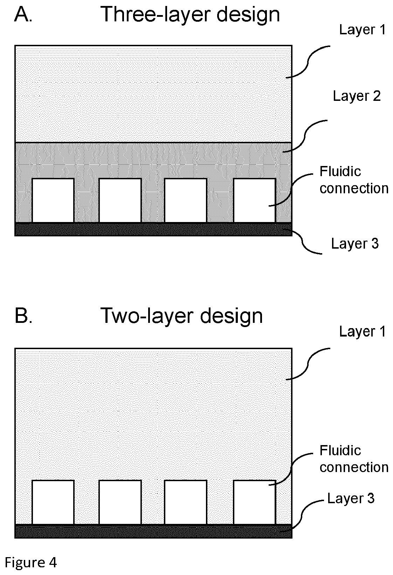

Furthermore, for assaying mechanisms relating to axonal transport, a multi-well plate can be designed in such a way that two non-adjacent wells have at least one fluidic connection and where said fluidic connections traverse a well adjacent to at least one of said non-adjacent wells to form a detection zone where the optical conditions are optimized for high resolution experiments using a high content imaging instrument (Figure 4 and 5). To facilitate autofocusing of the high-content imaging system, the wells can be equipped with optical detection markers in the same plane as the at least one fluidic connection. The cross-sectional dimensions of the fluidic connections may in this case comprise one or more connections having a dimension between 1-20 µm, such as 1-5 µm, 1-10 µm, 5-10 µm, 10-20 µm, 10-15 µm, 15-20 µm, 5-15 µm, or having a dimension (H or W) of 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, or 20 µm, and optionally also having an aspect ratio (H x W) ranging from 1:5 - 2:1. In some embodiments the length of the fluidic connection spanning one adjacent well is at least 3-5 mm.

In some embodiments the cross-sectional dimensions of the fluidic connections comprise less than 1 x 0.5 mm. In one embodiment, the fluidic connection is comprised of one or more connections having dimensions as small as 3 x 3 µm. The connections could also have larger dimensions, up to 100 x 100 µm. The aspect ratio, i.e., the ratio between width and height of cross-sectional dimensions could range from aspect ratios of 20:1 (W:H) to 1:5 (W:H), such as from 20:1 to 10:1, from 10:1 to 5:1, from 5:1 to 1:1, from 2:1 to 1:2, from 1:1 to 1:2, from 1:1 to 1:5, or from 1:2 to 1:5 (all W:H).

The shape and size of the fluidic connections may vary across the length-axis of the connection to optimize parameters such as producibility, fluid wetting and filling, and entrance of cellular processes into the fluidic connectors. For example, incorporation of funnel-like structures at the entrances of the fluidic connections can improve neurite guiding and penetration and varying the height of the fluidic connections can improve mechanical stability and thus producibility. In one embodiment, channels having 6 x 8 µm (W x H) dimensions are expanded to 20 x 8 µm (W x H) over a distance of 200 µm, thereby improving axon and dendrite guidance into the fluidic connection. In another embodiment, these funnel-like structures at the are joined together to form one large fluidic connection at the entrance, further improving neurite guidance and penetration. In one embodiment, this large fluidic connection at the entrance is also higher, significantly improving production yield of the multi-well plate. In one embodiment, the height of the fluidic connection is increased from 8 µm to 50 µm, but other heights can also be envisioned.

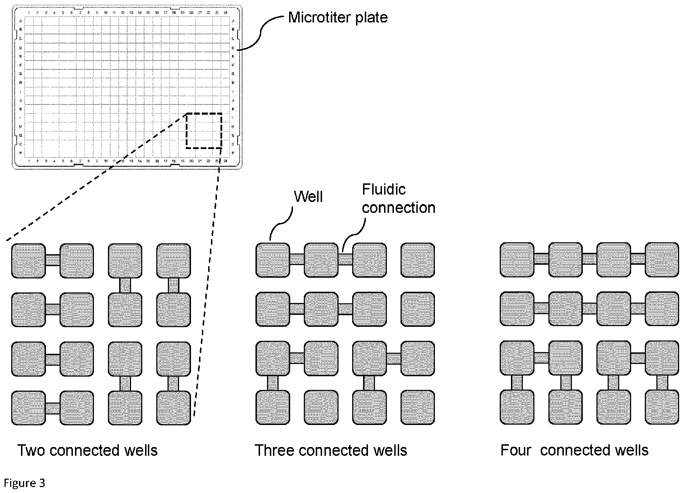

Also, depending on the assay application, the number of connected wells may vary. In one embodiment of the disclosure, the substrate contains several units of pair-coupled wells (i.e., two connected walls), in a second embodiment of the disclosure the substrate contains several units of three connected wells, and in a third embodiment of the disclosure, the substrate contains several units of four or more connected wells. (Fig. 3) In one embodiment of the disclosure, the fluidic connections are formed directly in the first layer of the substrate thus completely omitting the need to include a second layer in the substrate. (Fig. 4).

The third part of the substrate defines the bottom of the substrate. In order to enable high resolution imaging readouts, in some embodiments this bottom part of the substrate is optically transparent within the visible and far UV light spectra range and sufficiently thin to enable imaging using high numerical aperture microscope objectives. Accordingly, in some embodiments, the thickness of the third bottom part is less than 200 µm, such as 10-50 µm, 50-100 µm, or 100-200 µm. In other embodiments of the disclosure where high-resolution imaging is not utilized, the bottom layer of the substrate can be made thicker to increase mechanical robustness of the substrate. (Fig. 1). Accordingly, in some embodiments, the thickness of the third bottom part is in the range of 200-1000 µm, such as 200-500 µm, or 300-700 µm, or 500-1000 µm, or 200 µm, 300 µm, 400 µm, 500 µm, 600 µm, 700 µm, 800 µm, 900 µm, or 1000 µm.

In some embodiments, the wells in the substrate may be equipped with optical markers that resides in the same plane as the fluidic connections to enable microscopes and high-content imaging instruments to autofocus and subsequently localize the fluidic connections. Non-limiting examples of such optical markers are contrast enhancing prints or contrast enhancing microstructures.

### B. Methods of Substrate Production

The substrate of the present disclosure can be produced from a wide range of materials such as thermoplastics. Exemplary thermoplastic materials may include, for example, polystyrene (PS), cyclo-olefin-copolymer (COC) or cycloolefin polymer (COP), poly(methyl methacrylate (PMMA), polycarbonate (PC), polyethylene (PE), polyethylene terephthalate (PET), polyamide (Nylon®), polypropylene or polyether ether ketone (PEEK) Additional material groups may include perfluorinated materials like Teflon®, silicone polymers like PDMS, thermoset polymers such as thermoset polyester (TPE) or hard crystalline or amorphous materials, such as silicon, glass or ceramics such as alumina. However, to meet cost criteria for high-throughput screening where disposable substrates may be preferred, and large volumes of substrates may be consumed, the substrate may be produced from PS, COC or COP, as these materials may be amenable to cost-efficient high-volume production methods such as injection molding, hot embossing, or computer-aided manufacturing (CAM) micro machining. In some embodiments, the material to be used in the substrate is amenable for surface coatings to enable culture of cells. For example, it may be desirable in some embodiments to carry out physical surface treatments, e.g. plasma treatment or corona discharge, as well as to coat the substrate with materials proteins or polymeric materials such as poly-l-lysine, poly-L-ornithine, collagen, laminin, Matrigel®, bovine serum albumin or other protein solutions. Furthermore, chemical modifications can also be grafted onto the surface, in example poly[carboxybetaine methacrylate] (PCBMA), poly[[2-methacryloyloxy)ethyl]trimethylammonium chloride] (PMETAC), poly[poly(ethylene glycol) methyl ether methacrylate] (PPEGMA), poly[2-hydroxyethyl methacrylate] (PHEMA), poly[3-sulfopropyl methacrylate] (PSPMA), and poly[2-(methacryloyloxy)ethyl dimethyl-(3-sulfopropyl)ammonium hydroxide] (PMEDSAH).

To assemble the different layers of the substrate, a range of bonding methods can be utilized. For example, methods like ultrasonic welding, thermocompression bonding, plasma bonding, solvent-assisted bonding, laser-assisted bonding or adhesive bonding using glue or double adhesive tape can be used. Although not preferred from a manufacturing viewpoint, the substrate may be composed of different materials. In one embodiment of the disclosure, the bottom layer is composed of glass whereas the other layers are composed of thermoplastics or a silicone polymer material.

### EXAMPLES

### Example 1 - Assay to study spreading of neurodegenerative disease associated peptides (NDAP) in neuronal circuits.

Spreading of neurodegenerative disease associated peptides (NDAPs) within the brain is considered as one of the major pathological mechanisms in progressive neurodegenerative diseases, such as Alzheimer's and Parkinson's disease. In this concept, pathological soluble forms of NDAPs, such as amyloid-beta, alphasynuclein and tau proteins, are incorporated by neurons where they cause progression of protein misfolding, synapse elimination and neuronal cell loss. Moreover, a plethora of literature reports a prion-like mechanism of intracellular NDAPs, i.e. the intracellular transport of NDAPs and spreading from one neuron to another. Since neurodegenerative diseases are still virtually non-treatable, a high throughput assay platform that reflect all these complex neuropathological features in vitro and that allows screening and profiling of larger compound sets to prevent this neurodegenerative cascade, represents an urgent unmet clinical need.

Using the substrate of the present disclosure, we have been able to create a unique high throughput in vitro assay that reflects all hallmarks of the neurodegenerative disease cascade within CNS neuronal circuits. Mouse cerebral cortical neuronal cultures are being used since neurons in these cultures develop extensive processes and form functional synaptic connections in vitro.

In detail, mouse cortical E18 neurons were plated in a customized CCC substrate having a 384-well plate format containing 96 experimental units composed of three neighboring wells that were fluidically connected. In this application, it is of paramount importance that cells cannot migrate between neighboring wells, and therefore the fluidic connections were made smaller than a neuronal cell body. Therefore, each fluidic connection consisted of 10-30 holes with cross sectional diameters of 6 x 8 µm. Prior to seeding the cells, the substrate was coated first with a 0.01% poly-L-ornithine solution overnight at 37°C. The wells were thereafter washed with PBS with Ca²⁺/Mg²⁺ after which laminin diluted to 10 µg/ml in PBS with Ca²⁺/Mg²⁺ was added and incubated for 2 h at 37°C. Laminin was removed just prior to cell seeding. Cells were then prepared and cultured as described in Example 1. After 7 days in culture, the cells in zone 1's in all experimental units in the plate were treated with a 50 nM solution of NDAP polymers, e.g. patient-derived material such as pathogenic Tau protein oligomers extracted from the CSF of Alzheimer's patient. The plate was thereafter brought back into the incubator and the cells were cultured for 7 more days. After 14 DIV, the cells in the plate were fixed and stained for neuronal and assay-specific markers using immunocytochemical protocols, and high-content imaging is used to describe NDAP uptake, intraneuronal spreading and NDAP-mediated alteration of synapses and neuronal survival. Briefly, cells were fixed using 4% PFA in PBS or methanol. Neurons were evaluated using antibodies binding to mouse MAP-2AB (1:1000), chicken MAP-2AB (1:10000) or bTubIII (1:1000). Hoechst (nuclei) staining was also included. Anti-bTubIII (Sigma-Aldrich Sweden AB, Stockholm, Sweden) (1:1000), -PSD-95 (1:1000), -Synaptophysin (1:1000), -tau (1:1000), respectively, were combined with MAP-2AB antibodies (Sigma-Aldrich Sweden AB, Stockholm, Sweden). High-content imaging (HCA) analysis was performed using an Operetta® high content imager at 10x, 20x or 40x magnification (PerkinElmer).

To screen for modulators of spreading of NDAPs across synaptically coupled neurons, chemical, biologies- or genetic intervention can be performed in well two of the experimental unit to modulate the cell cultures ability to spread NDAPs, and well three of the unit is used to measure presence of NDAPs intracellularly that have spread through the cell-culture from well 1. An illustration of the assay concept is shown in Figure 7. Using this assay concept, we have demonstrated uptake and modulation of NDAPs. After 7 DIV, pathological Tau extracted from CSF of human AD patients was added as per above to the cells in zone's 1 in all experimental units. By balancing the liquid levels between the wells in the experimental units, it was ensured that no mass transport of Tau material took place between the wells in the experimental units. The pathological Tau was rapidly taken up by the cultures, and after 9 DIV, a modulating antibody was added to zone's 2 in all experimental units with the aim of modulating propagation of the Tau pathology in the culture. Again, liquid levels were balanced to ensure that no mass transport of antibody material took place between the wells in the experimental units. At 14-16 DIV, synaptic function was assessed in zone 3's in all experimental units in the plate by analyzing calcium fluorescence transients. Following this, cultures were fixed and stained for Beta tubulin type 3 and endogenous Tau (MAPT) and high-resolution images were acquired using a high content imager. Effects on synaptic function of the cultures together with effects on network integrity and endogenous Tau levels as analyzed by automated image analysis enabled high capacity screening for modulators of Tauopathy progression.

To our knowledge, this approach will show sufficient capacity and robustness to allow screening and profiling of larger compound sets in the search for molecules preventing spreading of NDAPs across synaptically coupled neurons

### Example 2 - Assay to monitor retrograde axonal transport in primary cortical neurons

The appearance and spreading of misfolded tau and α-synuclein (α-syn) proteins within patient brain cells are common pathological hallmarks in neurodegenerative diseases, e.g. Alzheimer's and Parkinson's disease. In vitro models have provided evidence for a neuron-to-neuron transfer of α-syn, where the transferred proteins act as seeds to cause aggregation of endogenous proteins in recipient neurons. This spreading of α-syn rely on several processes such as cellular uptake, axonal transport, somatic accumulation of seeds and aggregation of endogenous proteins. Here we have assessed α-syn retrograde axonal transport and the cellular consequences on primary neuronal cells using a novel microfluidic substrate.

### Assay Concept

The assay is based on a 384-well microtiter plate containing 96 experimental units. Each unit comprises of three spatially separated wells connected by microfluidic channels. The channels are sufficiently small to prevent migration of cells between the wells. We are using the co-culture plates for creating compartmentalized cell culturing and local interventions, in order to monitor and manipulate tau and α-syn axonal transport, somatic accumulation and neuronal pathologies (e.g. protein aggregation).

### Methods

To achieve compartmentalized neuronal cultures, mouse embryonic (E18) brain tissue-derived primary cortical cells were cultured only in one well of an experimental unit, while the interconnected wells were kept cell-free. After 6 days in vitro (DIV), either fluorescently labelled sonicated human α-syn fibrils (4 µg/ml, StressMarq Biosciences) were added to the cell-free wells. Well B have an excess volume of 50µl relative to well A in order to establish a hydrodynamic barrier. 7 days after application of seeds, plated were fixated and antibody-stained, imaged (PerkinElmer, Operetta), and quantitatively assessed.

### Results

Properties of compartmentalized neuronal cultures: Confocal images (20×) show that βTub3+, but MAP2AB-, axons grow through the microchannels to the interconnected well B while the MAP2AB+ neuronal soma and dendrites do not cross to well B. (Figure 9A). Through immunocytochemical staining we characterized the cellular composition of the cortical culture, and it is evident that the microfluidic substrate can maintain cultures that are identical to a standard microtiter plate. The cellular composition of the cortical cultures were as follows: ∼20.000 living cells per well, 90% NeuN+ neurons, and where 75% ±6% are PAX-6 +-excitatory neurons.

α-syn seeds are taken up by axons and are retrogradely transported to cells in well A: High content imaging show the localization of fluorescently labeled α-syn-633 seven days after the application of seeds. In the 20x images in figure 9B show fluorescently labeled α-syn-633 seed uptake by the axonal net in well B and the localization of fluorescently-labeled α-syn-633 seeds in the cortical cell population of well A. Detailed confocal images (40x) shows that the seeds are taken up by the axons in well B and are retrogradely transported towards the cell somata in well A.

Protein aggregation of endogenous proteins occur in α-syn seed-filled neurons: Seven days after application of α-syn seeds, confocal images demonstrates that NeuN+ neurons filled with fluorescently labeled α-syn seeds show phosphorylated endogenous α-synuclein (anti α-syn phosphorylated at Ser129, Abcam, clone [EP1536Y]) visible as neurite speckles and peri-nuclear accumulations (Figure 9C). Detailed confocal imaging showed colocalization of fluorescently labeled α-syn seeds and phosphorylated endogenous α-synuclein within NeuN+ neurons. Conclusion: We have established neuronal compartmentalization on our high capacity platform that enables monitoring of cellular processes involved in α-synucleinopathy. Using a microfluidic multi-well plate, we show that α-syn seeds are taken up by axons, retrogradely transported and accumulate in the soma of neurons, where they cause aggregation of endogenous proteins.

### Example 3 - Prophetic example - Assay for real-time monitoring of axonal transport processes in primary cortical neurons

There is evidence pointing to that defects in various transport processes in neurons, e.g. mitochondrial transport defects, are a contributing factor in neurodegenerative diseases, such as Parkinson's disease (Sterky et al, Proc. Natl. Acad. Sci. U. S. A. 108, 12937-12942 (2011)). This is further supported by the fact that this phenomenon can be reproduced and modulated in in-vitro disease models. However, MTS and/or HTS of mitochondrial transport processes along single axons are not feasible using traditional dissociated culture systems (Yu et al, Hum. Mol. Genet. 20, 3227-3240 (2011)). In this example we have assessed axonal transport in real-time using a novel microfluidic substrate along with automated live-imaging high content methods.

### Assay Concept

The assay is based on a 384-well microtiter plate containing 192 experimental units. Each unit comprises of two spatially separated wells where well 1 and 2 are connected by microfluidic channels (figure 4/5). In well 2 channels are predominantly enclosed with a small area accessible for microchannel access, and this serves primarily as an area of detection. The detection are used to trace organelles such as mitochondria or inserted tracer particles with the aim of being able to assess modulations of transport dynamics in the axons. Note that the channels are sufficiently small to prevent migration of cells between the wells, but that they will allow for penetration of axons, allowing for the detection transport processes in aligned axons only.

### Methods

Mouse embryonic (E16-18) brain tissue-derived primary cortical or midbrain cells can be cultured only in one well of an experimental unit, while the interconnected well is kept cell-free. When axons have sufficiently penetrated the channels, and when the cultures have reached a suitable level of maturation, transport dynamics can be traced in the axons of the live cells using automated time-lapse confocal microscopy to trace labelled endogenous structures or inserted particles. Depending on cell model and the level of desired maturation of the cultures, this assay can be carried out at after anywhere between 2-7 weeks in culture. In one example, transport dynamics in the axons are tracked by labeling mitochondria using a mitochondrial dye or fluorescent proteins using lentiviral particles and tracking the transport dynamics of these over a defined period. Typically, transport dynamics are tracked at 5 s intervals over a period of 3-5 minutes, after which e.g. transport velocities, the ratio of retro- to antero-grade transport etc. for the mitochondrial particles in the assay can be assessed. Using this methodology, modulation of transport dynamics in disease model as well as wild type cultures in MTS and HTS applications can be assessed.

### References

1. Campenot, R. B. Local control of neurite development by nerve growth factor. Proc. Natl. Acad. Sci. U. S. A. 74, 4516-9 (1977).
2. Vikman, K. S., Backström, E., Kristensson, K. & Hill, R. H. A two-compartment in vitro model for studies of modulation of nociceptive transmission. J. Neurosci. Methods 105, 175-184 (2001).
3. Bousset, L., Sourigues, Y., Covert, M. & Melki, R. Neuron-to-neuron transmission of α-synuclein fibrils through axonal transport. Ann. Neurol. 72, 517-524 (2013).
4. Taylor, A. M., Wu, J., Tai, H.-C. & Schuman, E. M. Axonal Translation of Catenin Regulates Synaptic Vesicle Dynamics. J. Neurosci. 33, 5584-5589 (2013).
5. Zahavi, E. E. et al. A compartmentalized microfluidic neuromuscular co-culture system reveals spatial aspects of GDNF functions. J. Cell Sci. 128, 1241-1252 (2015).
6. Berdichevsky, Y., Staley, K. J. & Yarmush, M. L. Lab Chip 10, 999-1004 (2010).). - Communication between different brain regions
7. Zhang, M., Luo, G., Zhou, Y., Wang, S. & Zhong, Z. J. Biomol. Screen. 19, 1-16 (2014 - Need for HTS prion-like mechanism platform
8. Anne M Taylor, Mathew Blurton-Jones, Seog Woo Rhee, David H Cribbs, Carl W Cotman, and N. L. J. A microfluidic culture platform for CNS axonal injury, regeneration and transport. Nat. Methods 2, 599-605 (2005).
9. Neto, E. et al. Compartmentalized Microfluidic Platforms: The Unrivaled Breakthrough of In Vitro Tools for Neurobiological Research. J. Neurosci. 36, 11573-11584 (2016).
10. ANSI/SLAS 1-2004 (R2012): Footprint Dimensions
11. ANSI/SLAS 2-2004 (R2012): Height Dimensions
12. ANSI/SLAS 3-2004 (R2012): Bottom Outside Flange Dimensions
13. ANSI/SLAS 4-2004 (R2012): Well Positions
14. ANSI/SLAS 6-2012: Well Bottom Elevation
15. Sterky, F. H., Lee, S., Wibom, R., Olson, L. & Larsson, N. G. Impaired mitochondrial transport and Parkin-independent degeneration of respiratory chain-deficient dopamine neurons in vivo. Proc. Natl. Acad. Sci. U. S. A. 108, 12937-12942(2011).
16. Lu, X., Kim-Han, J. S., O'Malley, K. L. & Sakiyama-Elbert, S. E. A microdevice platform for visualizing mitochondrial transport in aligned dopaminergic axons. J. Neurosci. Methods 209, 35-39 (2012).
17. Yu, W., Sun, Y., Guo, S. & Lu, B. The PINK1/Parkin pathway regulates mitochondrial dynamics and function in mammalian hippocampal and dopaminergic neurons. Hum. Mol. Genet. 20, 3227-3240 (2011).

## Claims

1. A multi-well plate comprising open wells having a optically transparent bottom and whose surface properties enables culturing of cells, wherein at least two wells in said plate are in direct fluidic connection through at least one closed microchannel of a sufficiently small size to prevent cell migration and to minimize cell and biomolecule diffusion between the wells, and where said at least one microchannel is located essentially in the plane of the bottom of the well.

2. The multi-well plate of claim 1, wherein a fluidic connection is formed between at least two adjacent wells.

3. The multi-well plate of claim 1, wherein a fluidic connection is formed between at least two non-adjacent wells and where the fluidic connection is traversing through the bottom of a well adjacent to at least one of the fluidically connected wells.

4. The multi well plate of claim 3 where in the plate further comprise optical detection markers in at least one well that is fluidically connected to another well.

5. The multi-well plate of claim 1, wherein the multi-well plate complies with American National Standards Institute of the Society for Laboratory Automation and Screening (ANSI/SLAS) microplate standards.

6. The multi-well plate of any one of the preceding claims, wherein the multi-well plate comprises a substrate produced from a thermoplastic material.

7. The multi-well plate of claim 6, wherein the thermoplastic material comprises polystyrene (PS), cyclo-olefin-copolymer (COC), cycloolefin polymer (COP), poly(methyl methacrylate (PMMA), polycarbonate (PC), polyethylene (PE), polyethylene terephthalate (PET), polyamide (Nylon®), polypropylene or polyether ether ketone (PEEK), Teflon®, PDMS, and/or thermoset polyester (TPE).

8. The multi-well plate of any one of the preceding claims, wherein the multi-well plate comprises a substrate produced from cyclo-olefin-copolymer (COP), cyclo-olefin-polymer (COC) or polystyrene (PS).

9. The multi-well plate of any one of the preceding claims, wherein the multi-well plate comprises a substrate produced from silicon, glass, ceramic material, or alumina.

10. The multi-well plate of any one of the preceding claims, wherein the plate comprises a substrate comprising more than one layer, optionally wherein the layers are bonded by ultrasonic welding, thermocompression bonding, plasma bonding, solvent-assisted bonding, laser-assisted bonding, or adhesive bonding using glue or double adhesive tape.

11. The multi-well plate of any one of the preceding claims, wherein the plate comprises a substrate coated with a protein or polymer.

12. The multi-well plate of claim 11, wherein the plate comprises a substrate coated with one or more of poly-l-lysine, poly-L-ornithine, collagen, laminin, Matrigel®, or bovine serum albumin.

13. The multi-well plate of any one of the preceding claims, wherein the plate comprises a substrate comprising a surface chemically modified with one or more of poly[carboxybetaine methacrylate] (PCBMA), poly[[2-methacryloyloxy)ethyl]trimethylammonium chloride] (PMETAC), poly[poly(ethylene glycol) methyl ether methacrylate] (PPEGMA), poly[2-hydroxyethyl methacrylate] (PHEMA), poly[3-sulfopropyl methacrylate] (PSPMA), and poly[2-(methacryloyloxy)ethyl dimethyl-(3-sulfopropyl)ammonium hydroxide] (PMEDSAH).

14. The multi-well plate of any one of the preceding claims, wherein the plate comprises at least two, at least four, at least 8, at least 16, at least 32, or at least 96 groups of three fluidically connected wells.

15. The multi-well plate of any one of the preceding claims, wherein the at least one fluidic connection comprises cross-sectional dimensions (H and/or W) between 1-20 µm, such as 1-5 µm, 1-10 µm, 5-10 µm, 10-20 µm, 10-15 µm, 15-20 µm, 5-15 µm, or comprising cross-sectional dimensions (H and/or W) of 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, or 20 µm, and optionally also having an aspect ratio (H x W) ranging from 1:5 - 2:1.

16. The multi-well plate of any one of the preceding claims, wherein the fluidic connection comprises cross-sectional dimensions of equal to or less than 5 x 5 µm, or of 3 x 3 µm to 5 x 5 µm.

17. The multi-well plate of claim 16, where the dimensions, shape and number of fluidic connections are varied across the length of the at least one fluidic connection to improve neurite penetration and producibility.

18. The multi-well plate of any one of the preceding claims, wherein the length of the at least one fluidic connection is at least 0.25 mm and at the most 5.0 mm.

19. The multi-well plate of any one of the preceding claims, wherein the aspect ratio of the dimensions of the at least one fluidic connection ranges from 20:1 (W:H) to 1:5 (W:H).

20. The multi-well plate of any one of the preceding claims, wherein the multi-well plate comprises a 96, 384, 1536 or 3456 well format, and is optionally organized in a 2:3 rectangular matrix.

21. The multi-well plate of any one of the preceding claims, wherein the multi-well plate comprises at least 2 groups of three neighboring and fluidically interconnected wells.

22. The multi-well plate of any one of the preceding claims, wherein the multi-well plate comprises at least 3 groups of two neighboring and fluidically interconnected wells.

23. The multi-well plate of any one of the preceding claims, wherein the multi-well plate comprises at least 1 group of four neighboring and fluidically interconnected wells.

24. A method for high throughput screening of a material of interest, comprising screening the material of interest using the multi-well plate of any one of claims 1 to 23.

25. The method of claim 24, wherein the material of interest is a 2D cell culture.

26. The method of claim 24, wherein the material of interest is a 3D cell culture.
